(19) Europäisches Patentamt
European Patent Office
Office européen des brevets

(11) **EP 1 871 466 B1**

(12) **EUROPÄISCHE PATENTSCHRIFT**

(45) Veröffentlichungstag und Bekanntmachung des Hinweises auf die Patenterteilung:
**03.06.2009 Patentblatt 2009/23**

(51) Int Cl.:
***A61N 1/36*** *(2006.01)*          ***A61F 9/08*** *(2006.01)*
***A61N 1/05*** *(2006.01)*

(21) Anmeldenummer: **06742568.6**

(22) Anmeldetag: **12.04.2006**

(86) Internationale Anmeldenummer:
**PCT/EP2006/003365**

(87) Internationale Veröffentlichungsnummer:
**WO 2006/108630 (19.10.2006 Gazette 2006/42)**

(54) **VORRICHTUNG ZUR ELEKTRISCHEN STIMULATION VON BIOLOGISCHEM MATERIAL**

DEVICE FOR ELECTRICALLY STIMULATING BIOLOGICAL MATERIAL

DISPOSITIF DE STIMULATION ELECTRIQUE DE MATIERE BIOLOGIQUE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **12.04.2005 DE 102005017740**

(43) Veröffentlichungstag der Anmeldung:
**02.01.2008 Patentblatt 2008/01**

(73) Patentinhaber: **Universität Tübingen**
**72074 Tübingen (DE)**

(72) Erfinder: **SCHMID, Em. Prof. Dr. Erich**
**72076 Tübingen (DE)**

(74) Vertreter: **Patentanwälte**
**Ruff, Wilhelm, Beier, Dauster & Partner**
**Postfach 10 40 36**
**70035 Stuttgart (DE)**

(56) Entgegenhaltungen:
**US-A- 4 628 933          US-A1- 2002 091 422**
**US-B1- 6 400 989**

- **RIZZO JOSEPH F 3RD ET AL: "Perceptual efficacy of electrical stimulation of human retina with a microelectrode array during short-term surgical trials." INVESTIGATIVE OPHTHALMOLOGY & VISUAL SCIENCE. DEC 2003, Bd. 44, Nr. 12, Dezember 2003 (2003-12), Seiten 5362-5369, XP002385700 ISSN: 0146-0404 in der Anmeldung erwähnt**

**Beschreibung**

Die Erfindung bezieht sich auf eine Vorrichtung zur elektrischen Stimulation von elektrisch stimulierbarem biologischem Material, insbesondere von Netzhautmaterial, nach dem Oberbegriff des Anspruchs 1.

Vorrichtungen dieser Art sind in verschiedenen Ausprägungen bekannt. Ein Anwendungstyp, der in jüngerer Zeit Gegenstand intensiver Entwicklung ist, sind sogenannte implantierbare Sehprothesen bzw. Retina-Implantate. Diese beinhalten typischerweise ein subretinal oder epiretinal in Wirkkontakt mit elektrisch stimulierbarem Netzhautgewebe implantierbares Elektrodenarray, das wenigstens in gewissem Maß einen zur Erblindung führenden Ausfall von Netzhaut-Photorezeptoren kompensieren soll, d.h. deren Funktion zur ortsaufgelösten Elektrostimulation von Netzhautnervenzellen ersetzen soll.

Am weitesten verbreitet sind hierzu unipolar stimulierende Elektrodenarrays, auch als monopolarer Typ bezeichnet, bei denen ein Elektrodenarraychip auf einer Vorderseite, die im implantierten Zustand dem stimulierbaren Netzhautgewebe zugewandt ist, eine zweidimensionale Anordnung von einzelnen Stimulationselektroden trägt, denen eine flächige Gegenelektrode in relativ weitem Abstand vor der Chipvorderseite oder an der Chiprückseite zugeordnet ist, d.h. in einem Abstand, der sehr viel größer als der Abstand benachbarter Stimulationselektroden ist. Die Stimulationselektroden können durch entsprechende Mittel mit einem Stimulationssignal beaufschlagt werden, typischerweise einem gepulsten Spannungssignal, durch das sie im benachbarten stimulierbaren Netzhautgewebe ein Stimulationsfeld erzeugen, typischerweise in Form elektrischer Ströme zwischen den Stimulationselektroden einerseits und der Gegenelektrode andererseits. Bei einem passiven Arraytyp wird die zur Stimulation nötige Energie z.B. durch eine Photodiodenschichtfolge im Arraychip allein dem einfallenden sichtbaren Licht entnommen. Zur Erzielung höherer Stimulationsströme bei gleichzeitig möglichst guter Ortsauflösung und folglich kleinflächigen Stimulationselektroden wird bei einem aktiven Arraytyp zusätzlich Energie eingekoppelt, z.B. durch gepulste Infrarotstrahlung. Zu diesen und weiteren Details unipolarer Elektrodenarrays sei auf die diesbezügliche Literatur verwiesen, siehe auch die Offenlegungsschriften DE 101 51 650 A1 und DE 103 29 615 A1 sowie die Patentschrift DE 197 05 987 C2.

Um für solche Retina-Implantate gleichzeitig eine zufriedenstellende Ortsauflösung und Ansprechempfindlichkeit zu erreichen, ist grundsätzlich ein Elektrodenarray mit einer sehr dichten Anordnung kleinflächiger Einzelelektroden wünschenswert, die in der Lage sind, ein hohes Stimulationsfeld, d.h. über einem Anregungsschwellwert der Netzhautzellen liegende Stimulationsströme, zu liefern und ein großes Stimulationsvolumen zu erfassen, in welchem das Stimulationsfeld über dem Anregungsschwellwert der Netzhautzellen liegt. Andererseits müssen lokal zu hohe Stimulationsfelder vermieden werden, die zu Gewebeschädigungen führen könnten.

Hier scheinen die herkömmlichen unipolaren Elektrodenarrays an Grenzen zu stoßen. Die Tatsache, dass bei Spannungsbeaufschlagung der Stimulationselektroden nur transiente Ströme beobachtet werden, wird der Bildung einer Helmholtz-Doppelschicht an der Grenzfläche von Elektrodenarray und Netzhautgewebe zugeschrieben, wenngleich die diesbezüglichen Wirkmechanismen noch nicht im einzelnen geklärt sind. Von einer porösen Gestaltung der Stimulationselektroden mit entsprechend erhöhter Elektrodenoberfläche wird die Erzielung erhöhter transienter Ströme berichtet, siehe z.B. den Zeitschriftenaufsatz A. Stett und H. Hämmerle, Subretinale Sehprothesen für Blinde, Bulletin SEVNSE 3/99, S. 11. Dies spricht eher gegen eine Betrachtung der Elektrodenarray-Netzhautgewebe-Grenzfläche als reiner elektrischer Kondensator und deutet auf "Batterieeffekte" durch elektrolytische Eigenschaften des Gewebes hin. Bei dichterer Packung der Stimulationselektroden und höheren Stromdichten ist zudem für die unipolaren Elektrodenarrays ein kollektiver Stromeffekt zu erwarten, der in unerwünschter Weise zu hohen Spannungsabfällen und dazu führen kann, dass die zur Stimulation anzulegende Spannung davon abhängt, wie viele Stimulationselektroden aktiv sind, d.h. ob die Sehumgebung eher hell oder dunkel ist.

Alternativ zu monopolaren Elektrodenarrays wurde auch schon mit bipolaren Elektrodenarrays experimentiert, bei denen den vorderseitigen Stimulationselektroden individuell vorderseitige Gegenelektroden zugeordnet sind. Einer solchen bipolaren Anordnung wird durch die lokale Begrenzung der Stromausbreitung im Netzhautgewebe die Fähigkeit zugesprochen, dass im Bereich zwischen den Elektroden stärkere Spannungsabfälle als beim monopolaren Typ erzeugt werden können, wie von A. Stett und H. Hämmerle im obigen Zeitschriftenaufsatz erwähnt. In dem Zeitschriftenaufsatz J.F. Rizzo III et al., Methods and perceptual thresholds for short-term electrical stimulation of human retina with microelectrode arrays, Investigative Ophthalmology & Visual Science, Dezember 2003, Band 44, Nr. 12, S. 5355 ist außer monopolaren Elektrodenarrays auch ein bipolares Elektrodenarray mit kreisrunden vorderseitigen Stimulationselektroden offenbart, die über einen Ringspalt von einer vorderseitigen zusammenhängenden Gegenelektrode getrennt sind.

Darüber hinaus ist aus der Ottenlegungschrift US 2002/091422 ein Retinastimulationssystem mit tripolaren oder multipolaren Elementarzellen eines Elektrodenarrays und Wechselfeldstimulation bekannt.

Der Erfindung liegt als technisches Problem die Bereitstellung einer Vorrichtung der eingangs genannten Art zugrunde, mit der sich ein elektrisch stimulierbares biologisches Material unter Verwendung eines implantierbaren Elektrodenarrays auch bei hoher Elektrodenintegrationsdichte mit vergleichsweise hohem Stimulationswirkungsgrad elektrisch stimulieren lässt.

Die Erfindung löst dieses Problem durch die Bereitstellung einer Vorrichtung mit den Merkmalen des Anspruchs 1. Diese Vorrichtung ist darauf ausgelegt, die Einzelelektroden des in Wirkkontakt zu dem elektrisch stimulierbaren biologischen Material implantierbaren Elektrodenarrays mit Wechselfeld-Stimulationssignalen derart zu beaufschlagen, dass das Elektrodenarray wenigstens zwei tripolare oder höher multipolare Multipol-Elementarzellen aus jeweils drei oder mehr benachbarten Einzelelektroden bildet, wobei das von jeder Multipol-Elementarzelle für das biologische Material erzeugte Stimulationsfeld, wie in Form elektrischer Stimulationsströme, eine Rotationskomponente aufweist und wenigstens eine der Einzelelektroden zu wenigstens zwei Multipol-Elementarzellen gehört. Damit ist es möglich, ein Gesamtstimulationsfeld aus einzelnen Multipolfeldern der Elementarzellen mit rotierender Feldkomponente zu erzeugen, wobei die Feldkomponenten je zweier benachbarter Multipol-Elementarzellen z.B. gleichzeitig und gegensinnig rotieren können.

Die Rotation des Stimulationsfeldes ermöglicht aufgrund ihres "Leuchtturmeffektes" eine deutliche Steigerung der Wahrscheinlichkeit, dass im benachbarten biologischen Material eine stimulierbare Zelle von einem über deren Anregungsschwellwert liegenden lokalen Stimulationsvektor "getroffen" und dadurch tatsächlich stimuliert wird. Hierbei ist zu berücksichtigen, dass im allgemeinen und speziell auch in der Retinaanwendung die stimulierbaren Zellen im biologischen Material relativ unregelmäßig in einem Netzwerk angeordnet sind und zudem ihr Anregungsschwellwert meist richtungsabhängig ist. So wird bei den zylinder/stabförmigen Axonen und Dendriten der Netzhaut beobachtet, dass sie parallel zu ihrer Zylinderrichtung leichter, d.h. mit geringerem Anregungsschwellwert, stimuliert werden als in dazu senkrechter Richtung. Durch die Rotation des Stimulationsfeldes erhöht sich die Wahrscheinlichkeit, dass eine stimulierbare Zelle selbst in einer ungünstigeren Stimulationsrichtung von einem genügend hohen Stimulationsfeldvektor oder wenigstens noch in einer günstigen Stimulationsrichtung mit dann niedrigerem Anregungsschwellwert von einem Stimulationsfeldvektor getroffen wird. In beiden Fällen wird die Zelle dann stimuliert, während sie bei nicht rotierendem Stimulationsfeld eventuell nicht mit einem genügend großen Anregungsfeldvektor getroffen und daher nicht stimuliert würde.

Mit anderen Worten steigert die erfindungsgemäße Rotation des Stimulationsfeldes den Stimulationswirkungsgrad und das Stimulationsvolumen, d.h. das Volumen des vom Stimulationsfeld stimulierbaren biologischen Materials, was besonders bei sehr kleinen Elektrodenabständen von Bedeutung ist. Der Rotationskomponente können je nach Anwendungsfall weitere Feldkomponenten überlagert sein, z.B. eine zeitlich pulsierende Komponente und/oder eine Oszillationskomponente, d.h. eine in zwei entgegensetzten Richtungen oszillierende Komponente.

In vorteilhafter Weiterbildung der Erfindung werden die drei oder mehr benachbarten, zu einer entsprechenden Multipol-Elementarzelle, d.h. als Tripol, Quadrupol etc., gekoppelten Einzelelektroden derart mit dem Wechselfeld-Stimulationssignal beaufschlagt, dass das von ihnen gemeinsam im biologischen Material erzeugte Stimulationsfeld eine um eine Multipolachse rotierende Feldkomponente aufweist. Diese Multipol-Ansteuerung der Einzelelektroden ist eine besonders einfach realisierbare und wirksame Möglichkeit der Erzeugung eines Stimulationsfeldes mit Rotationskomponente. So kann in weiterer Ausgestaltung der Erfindung ein Stimulationsfeld mit einer mit konstanter Winkelgeschwindigkeit rotierenden Feldkomponente dadurch erzeugt werden, dass die Einzelelektroden des Multipols mit gegeneinander geeignet phasenverschobenen Wechselspannungssignalen beaufschlagt werden. Gleichzeitig kann eine Oszillationskomponente erzeugt werden. Durch die Kopplung der Einzelelektroden zu einem oder mehreren Multipolen wird ein entsprechend multipolares Elektrodenarray gebildet, bei dem jede Einzelelektrode des oder der Multipole sowohl als Stimulationselektrode als auch als Gegenelektrode im herkömmlichen Sinn wirkt, d.h. die Einzelelektroden können sich alle auf der im implantierten Zustand dem biologischen Material zugewandten Vorderseite des Elektrodenarrays befinden, und das mindestens tripolare Elektrodenarray bildet eine höherpolare Erweiterung der oben erwähnten monopolaren und bipolaren Elektrodenarrays.

In Weiterbildung der Erfindung bilden die Einzelelektroden des Elektrodenarrays eine vorzugsweise regelmäßige Elektrodengitteranordnung aus mehreren nebeneinander liegenden Multipol-Elementarzellen, die jeweils wenigstens drei Einzelelektroden umfassen, wobei jede innere Elektrode des Gitters zu mehreren Multipol-Elementarzellen gehört.

In weiterer Ausgestaltung der Erfindung bilden die Einzelelektroden z.B. ein regelmäßiges Dreieckgitter, bei dem je drei benachbarte Einzelelektroden zu einem Tripol gekoppelt werden können, oder ein regelmäßiges Viereckgitter, bei dem je vier benachbarte Einzelelektroden zu einem Quadrupol gekoppelt werden können, wobei die erzeugten Stimulationsfelder von je zwei benachbarten Multipolen gleichzeitig gegensinnig rotierende Komponenten aufweisen können.

In Ausgestaltung der erfindungsgemäßen Maßnahme, die Einzelelektroden einer Multipol-Elementarzelle mit phasenverschobenen Wechselspannungssignalen zu beaufschlagen, werden als zusätzliche Faktoren ein Bildinformationsanteil und ein Signalformanteil berücksichtigt, die für die Einzelelektroden individuell gewählt werden können. Der Bildinformationsanteil enthält die wahrzunehmende Bildinformation, der Signalformanteil enthält zusätzliche Signalformungsmaßnahmen, wie eine gepulste Ansteuerung mit vorgebbaren Pulsparametern.

In einer speziellen vorteilhaften Ausgestaltung stellt die Erfindung ein retina-implantierbares Mikroelektrodenarray zur Verfügung, bei dem die Einzelelektroden sämtlich auf einer Seite eines Trägerchips angeordnet sind und auf eine der oben erwähnten Arten als Tripole oder höhere Multipole gekoppelt angesteuert werden, so dass ein Multipol-Stimulationsfeld mit rotierenden Feldbeiträgen der einzelnen Multipole zur Netzhautstimulation erzeugt wird, wobei die Feldbeiträge benachbarter Multipole gleichzeitig und gegensinnig rotieren können.

Vorteilhafte Ausführungsformen der Erfindung sind in den Zeichnungen dargestellt und werden nachfolgend beschrieben. Hierbei zeigen:

Fig. 1 eine schematische, ausschnittweise Draufsicht auf ein z.B. für ein Retina-Implantat verwendbares Multielektrodenarray mit Dreieckgitteranordnung der Einzelelektroden,

Fig. 2 eine Draufsicht auf die Anordnung von Fig. 1 in einem Tripol-Ansteuerbetrieb mit gegensinnig rotierenden Tripol-Stimulationsstromvektoren,

Fig. 3A bis 3F Momentandarstellungen der Amplitude und Polarität von Ansteuerwechselspannungen und Ansteuerfeldvektorrichtungen für den Ansteuerbetrieb gemäß Fig. 2 zu verschiedenen Zeiten einer Wechselspannungsperiode,

Fig. 4 eine Ansicht entsprechend Fig. 1 für ein Multielektrodenarray mit Rechteckgitteranordnung der Einzelelektroden,

Fig. 5 eine Ansicht entsprechend Fig. 2 für einen Quadrupol-Ansteuerbetrieb des Elektrodenarrays von Fig. 4 und

Fig. 6A bis 6H Momentandarstellungen entsprechend den Fig. 3A bis 3F für den Quadrupol-Ansteuerbetrieb gemäß Fig. 5 zu verschiedenen Zeitpunkten einer Wechselspannungsperiode.

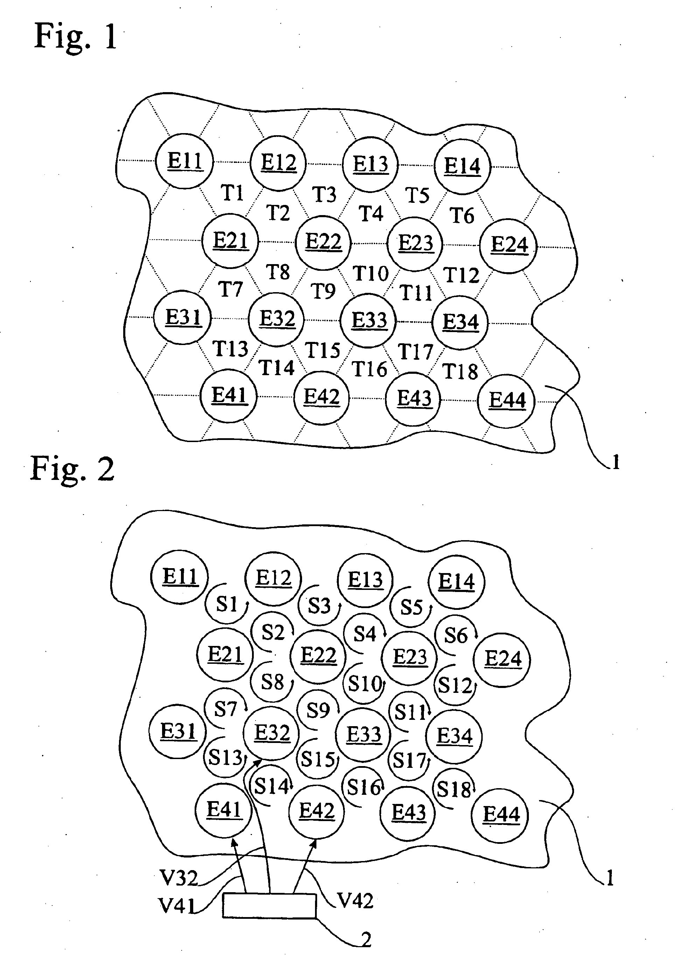

In Fig. 1 ist schematisch und ausschnittsweise ein Mikro-/Multielektrodenarray 1 in Draufsicht gezeigt, das eine Anzahl n·m von Einzelelektroden Eij, mit i = 1, 2, ..., n und j = 1, 2, ..., m, aufweist, die in einer regelmäßigen Dreieckgitterstruktur angeordnet sind, wie durch die Dreieckgitterlinien zwischen den Elektroden Eij symbolisiert. Dabei sind n und m beliebige, vorzugsweise relativ große natürliche Zahlen, z.B. zwischen 10 und 1000. Soweit nachfolgend nichts weiteres gesagt ist, ist das Elektrodenarray 1 im übrigen von irgendeinem der herkömmlichen, in Wirkkontakt mit einem elektrisch stimulierbaren biologischen Material implantierbaren Typen. Insbesondere ist es als Retina-Implantatchip zur subretinalen oder epiretinalen Implantation realisierbar.

Das Elektroden-Dreieckgitter beinhaltet alle benötigten Elektroden, d.h. die Stimulationselektroden und die Gegenelektroden, wobei jede Elektrode Eij als Stimulations- und Gegenelektrode fungiert. Speziell sind je drei benachbarte Elektroden zu einer Tripol-Elementarzelle zusammengefasst, d.h. sie werden als Tripol gekoppelt angesteuert, z.B. die Elektroden E11, E12 und E 21 als ein erster Tripol T1, die Elektroden E12, E22 und E21 als ein zweiter Tripol T2 etc., wobei die Tripolbezeichnungen T1, T2, ... jeweils in der zugehörigen Dreieckmitte eingetragen sind. Da im Dreieckgitter jeder Punkt sechs nächste Nachbarn besitzt, gehört jede Einzelelektrode Eij sechs Tripolen an. Dabei kann jeweils ein Tripol T1, T2, ... als ein Bildpunkt bzw. Pixel z.B. eines Retina-Implantats verstanden werden.

Durch geeignet gekoppelte Ansteuerung der Einzelelektroden Eij mit phasenverschobenen Wechselspannungssignalen ist es möglich, mit dem Dreieckgitter ein kollektives Stimulationsstromfeld mit Feldbeiträgen S1, S2, ... der einzelnen Tripole T1, T2, ... zu erzeugen. Die einzelnen Tripol-Stromfelder S1, S2, ... weisen, wie in Fig. 2 angedeutet, eine Rotationskomponente um eine jeweilige Tripolachse und damit um eine zur lokalen Normalenrichtung des Elektrodenarrays 1 parallele Achse auf, die in der Mitte des jeweiligen Tripols T1, T2, ... am stärksten ist und zu den Tripolelektroden hin abnimmt. Gleichzeitig weisen sie eine Oszillationskomponente auf, die über den Tripolelektroden am stärksten ist und zur Tripolmitte hin abnimmt. Um einen solchen Ansteuerbetrieb zu bewirken, werden die Einzelelektroden Eij individuell mit einer zeitabhängigen Ansteuerspannung Vij(t) der Form

$$V_{ij}(t) = I_{ij}(t) \cdot B_{ij}(t) \cdot \sin(\omega t + M_{ij})$$

angesteuert, wobei Iij(t) eine zeitabhängige Bildmatrix, Bij(t) eine zeitabhängige Signalformmatrix und Mij eine Phasenmatrix bezeichnet, die im vorliegenden Beispiel einer Tripolanordnung wie folgt gewählt werden kann:

$$M_{ij} = (2\pi/3) \cdot S_{ij} + \pi/2,$$

mit folgender Matrix Sij:

$$Sij = \begin{pmatrix} 0 & 1 & 2 & 0 & . \\ 2 & 0 & 1 & 2 & . \\ 0 & 1 & 2 & 0 & . \\ 2 & 0 & 1 & 2 & . \\ . & . & . & . & . \end{pmatrix}$$

Die Phasenmatrix Mij gibt folglich die Phasenverschiebung des Ansteuerspannungssignals Vij zwischen den Einzelelektroden Eij an. Ersichtlich wechselt die Phase der Ansteuerspannung Vij aufgrund der Phasenmatrix Mij zwischen je zwei Elektroden eines jeweiligen Tripols T1, T2, ... um $2\pi/3$(modulo $\pi$), wie im Fall einer üblichen dreiphasigen Wechselspannung eines Drehstromnetzes.

Zusätzlich kann die Zeitabhängigkeit des Ansteuersignals Vij(t) für jede Einzelelektrode Eij durch die Bildmatrix Iij(t) und die Signalformmatrix Bij(t) multiplikativ gemäß der obigen Beziehung beeinflusst werden, wie dies an sich für die Ansteuerung von Stimulationselektroden in Retina-Implantaten bekannt ist, worauf verwiesen werden kann. Insbesondere repräsentiert die Bildmatrix Iij die ortsaufgelöste, wahrzunehmende Bildinformation, wie sie z.B. von einer elektronischen Bildaufnahmeeinheit geliefert wird, bei der es sich insbesondere auch um eine Photodiodenanordnung einer für Retina-Implantate üblichen Art handeln kann. Die Signalformmatrix Bij, auch mit dem englischen Ausdruck "beat matrix" genannt, dient dazu, eine für den jeweiligen Anwendungsfall geeignete Signalform für die Ansteuersignale Vij einzustellen, z.B. ein gepulstes Signal mit vorgebbaren Pulsparametern, wie Pulsamplitude und Pulsdauer, wobei ein Spannungspuls vorzugsweise von deutlich kürzerer Dauer gewählt wird als die Tripol-Rotationsperiode $\tau=(2\pi/\omega)$.

Im realen Fall werden durch entsprechende Wahl der Signalformmatrix mit den Elementen Bij(t) geeignete Signalformen gewählt, z.B. solche, die experimentell erprobt sind oder aus anderen Gründen als tauglich angesehen werden. Hierzu gehören Kurzzeitpulse von weniger als einer Millisekunde Dauer, z.B. im Rechteckform. Die Wiedergabe solcher Kurzzeitpulse durch Bij(t), in Verbindung mit einer Rotationsperiode $\tau=(2\pi/\omega)$ von z.B. in der Größenordnung von 20 Millisekunden, führt dann dazu, dass das Stimulationssignal während der Rotation nur in einem schmalen Winkelbereich auftritt, der als "Schussrichtung" bezeichnet werden kann. Es gibt dann grundsätzlich zwei Möglichkeiten. Bei der ersten Möglichkeit ändert sich die Schussrichtung bei wiederholter Aussendung des Stimulationssignals. Von dieser Möglichkeit kann z.B. Gebrauch gemacht werden, wenn eine relativ schonende Stimulation, d.h. geringe Werte der den Elektroden beaufschlagten Spannungen, gewünscht wird, bei der aber noch Nervenzellen "getroffen", d.h. stimuliert werden. Bei der zweiten Möglichkeit ändert sich die Schussrichtung nicht bei wiederholter Aussendung des Stimulationssignals für jeden einzelnen Multipol. Sie ändert sich nur in vorgebbarer Weise beim Übergang zu anderen Multipolen des Arrays. Von dieser zweiten Möglichkeit kann z.B. Gebrauch gemacht werden, wenn es Nervenzellen oder Teile von solchen gibt, die nicht oder nur mit verringerter Wahrscheinlichkeit stimuliert werden sollen. Hierzu gehören z.B. bei epiretinaler Anwendung die durchlaufenden Axone, welche die Information von weiter entfernt liegenden Gesichtsfeldarealen zum Sehnerv leiten. Auf diese Weise wird der zusätzliche Freiheitsgrad, der durch die Feldrotation in Verbindung mit der Signalform gewonnen wird, zur Gestaltung der Stimulationssignale genutzt.

Im Ergebnis führt das Ansteuersignal Vij der oben erläuterten Art für die Einzelelektroden Eij zu einem Stimulationsstromfeld, das sich aus den einzelnen Tripol-Stimulationsstromfeldern der Tripole T1, T2, ... zusammensetzt, die jeweils mit der Bildinformation Iij und der typischerweise gepulsten Signalform Bij moduliert sind. Zur Bereitstellung der individuellen Ansteuersignale Vij für die Einzelelektroden Eij dienen beliebige, zu diesem Zweck an sich bekannte Mittel, die in Fig. 2 lediglich schematisch als Ansteuerspannungs-Bereitstellungseinheit 2 dargestellt sind, wobei in Fig. 2 stellvertretend für die Ansteuerung aller Einzelelektroden Eij nur diejenige für die drei Elektroden E32, E41 und E42 des Tripols T14 explizit gezeigt ist. Die Einheit 2 kann sich ganz oder teilweise auf dem Arraychip 1 befinden und je nach Anwendungsfall vom passiven oder aktiven Typ sein, d.h. allein von einfallendem sichtbarem Licht oder von zusätzlich zugeführter Fremdenergie gespeist werden. Zu der Einheit 2 können insbesondere übliche, nicht näher gezeigte Photodiodenelemente zur ortsaufgelösten Erfassung der wahrzunehmenden Bildinformation gehören.

Fig. 2 veranschaulicht den vereinfachten Fall der Betrachtung einer "weißen Wand", d.h. Iij(t)=1 für alle i und j und konstant über die betrachtete Zeit t, und ohne zusätzliche Signalformung, d.h. Bij(t)=1 für alle i, j und t. Wie unmittelbar aus den obigen Beziehungen ersichtlich, ergibt sich dann eine reine Tripol-Spannungsansteuerung der Elektroden Eij.

In der schematischen Draufsicht von Fig. 2, d.h. in Projektion auf die Elektrodenarrayebene, weisen die einzelnen Tripol-Stimulationsstromfelder S1, S2, ... eine Komponente auf, die mit der Winkelgeschwindigkeit $\omega$ der Ansteuerwechselspannung Vij um die jeweilige Tripolachse rotiert, d.h. um eine lokal zum Elektrodenarray 1 senkrechte Achse.

Diese Rotationskomponente ist in bzw. über der Tripolmitte am stärksten, wo die Feldlinien eine horizontale, d.h. zur Elektrodenarrayebene parallele Tangente besitzen, und nimmt zu jeder der drei beteiligten Elektroden hin ab wie für ein Tripol-Wechselfeld an sich bekannt. Die Rotationskomponenten jeweils übernächster Tripole rotieren gleichsinnig im uhrzeigersinn bzw. Gegenuhrzeigersinn, d.h. die Rotationskomponenten jeweils benachbarter Tripole rotieren gegensinnig. Gleichzeitig weisen die Tripol-Stromfelder, was hier der Einfachheit halber nicht explizit dargestellt ist, eine Oszillationskomponente auf, die an bzw. über den Elektroden am stärksten ist und zur Tripolmitte hin abnimmt.

Zu beachten ist auch, dass wegen der Lade/Entlade-Charakteristik der Elektroden zwischen der Rotation des beaufschlagten Spannungsfeldes und der Rotation des Stimulationsstromfeldes in der Regel ein zeitlicher Gangunterschied besteht. In linearisierter Näherung für die Lade/Entlade-Charakteristik, d.h. in Kondensatornäherung, kann dieser Gangunterschied analytisch berechnet werden, wie allgemein bekannt. Weiter ist zu beachten, dass die gezeigten Ströme für ein als Modell angenommenes elektrisch homogenes biologisches Material gelten.

Wenngleich dies somit nur eine qualitative Abschätzung eines idealisierten Falles darstellt, ergibt sich auch für den allgemeinen realen Fall, dass die Stimulationsstromvektoren S1, S2, ... in jedem Tripolgebiet T1, T2, ... des Elektroden-Dreieckgitters nach Festlegung einer jeweils geeigneten Ansteuerspannung Vij(t) vorzugsweise durch eine Rechnereinheit, z.B. als Teil der Ansteuerspannungs-Bereitstellungseinheit 2, eine Rotationskomponente um die Tripolachse mit gegensinniger Rotationsrichtung der Stimulationsstromvektoren benachbarter Tripole und eine dazu überlagerte Oszillationskomponente aufweisen.

Die Rotationskomponente realisiert einen "Leuchtturmeffekt" des Stimulationsstromfeldes, d.h. in jedem Tripolgebiet T1, T2, ... gibt es rotierende Stimulationsstromvektoren S1, S2, ..., wie dies in der Projektion auf die Elektrodenarrayebene in Fig. 2 schematisch dargestellt ist. Diese mit der Grundfrequenz ω der elektrodenansteuernden Wechselspannung Vij(t) rotierenden Stimulationsstromvektoren S1, S2, ... rastern zeitabhängig ein gewisses Stimulationsvolumen mit unterschiedlichen Vektorrichtungen ab.

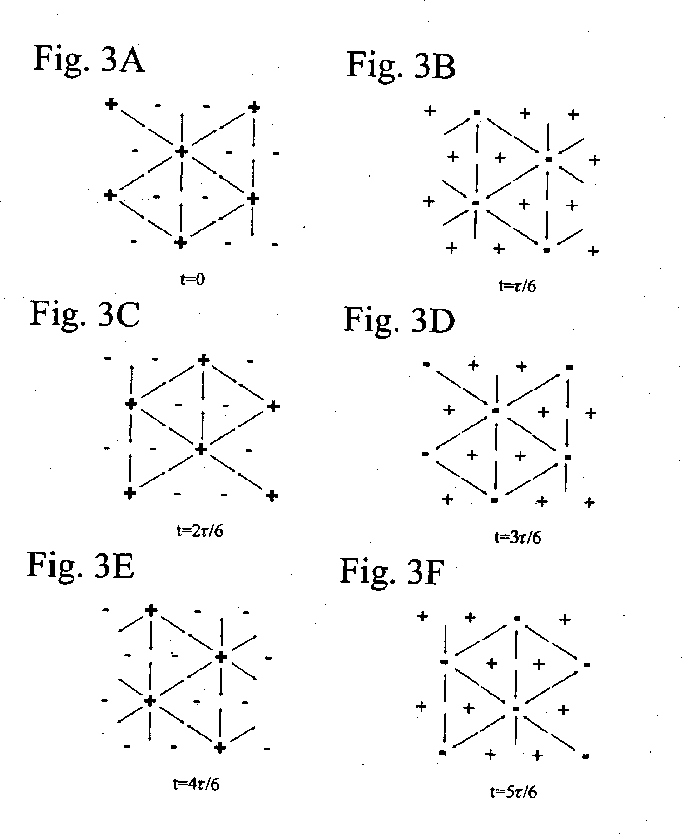

Die Fig. 3A bis 3F veranschaulichen in selbsterklärender Weise detaillierter die Entstehung der rotierenden Stimulationsvektorkomponente durch diese spezielle Tripol-Wechselspannungsansteuerung der Elektroden, wobei der Einfachheit halber für diese Darstellung die Elemente der Bildmatrix Iij und der Signalformmatrix Bij gleich eins gesetzt sind. Dazu ist in Abständen $\Delta t = \tau/6$ von einem Sechstel der Periode $\tau = 2\pi/\omega$ der ansteuernden Wechselspannung in einem jeweiligen Momentandiagramm für jeden Dreieckgitterpunkt von Fig. 2, d.h. jede Elektrode Eij, die Polarität "+" bzw. "-" der momentan anliegenden Spannung dargestellt, wobei eine höhere Amplitude mit dicker bezeichnetem Polaritätszeichen repräsentiert ist. Zusätzlich sind die momentanen horizontalen Vektorkomponenten des angelegten elektrischen Feldes qualitativ als Pfeile angegeben, die sich für den jeweiligen Zeitpunkt in der Mitte jedes Tripolgebietes ergeben. Aus der zeitlichen Abfolge der Diagramme der Fig. 3A bis 3F wird deutlich, dass und wie sich die zur Arrayoberfläche parallele Komponente des angelegten Feldes im Mittenbereich jedes Tripolgebietes mit der Frequenz der ansteuernden Wechselspannung dreht. Im realen Fall ist noch der zeitabhängige Einfluss durch die Bildmatrix Iij(t) und die Signalformmatrix Bij(t) überlagert.

Die Bereitstellung eines rotierenden Stimulationsstromfeldes bedeutet gerade auch in der Anwendung als Retina-Implantat eine signifikante Erhöhung der Wahrscheinlichkeit, dass innerhalb eines gegebenen Netzhautgewebevolumens ein Stimulationsstromvektor in seiner Stärke und Richtung eine Netzhautzelle so trifft, dass ihr Anregungsschwellwert überschritten wird, so dass in ihr ein Nervenreiz ausgelöst wird, der als ortsaufgelöste Bildinformation weitergeleitet werden kann. Dabei ist zu berücksichtigen, dass die Ansprechempfindlichkeit der Axonen und Dendriten des Retinagewebes, die typischerweise von zylindrischer Struktur sind, richtungsabhängig unterschiedlich ist.

Dies erhöht folglich den Stimulationswirkungsgrad. Außerdem erhöht die Rotation des Stimulationsstromfeldes das Stimulationsvolumen, da auch noch Zellen in einer größeren Entfernung mit einem ausreichenden Stimulationsstrom getroffen werden können, wenn sich dieser in eine günstige Richtung gedreht hat. Dazu trägt auch bei, dass sich ein Teil der Feldlinien des Tripolfeldes ebenso wie jedes anderen höheren Multipolfeldes von der Elektrodenarrayoberfläche aus mit noch relevanter Anregungsstromstärke merklich von der Arrayoberfläche entfernt und bogenförmig wieder zu ihr zurückkehrt. Zusätzlich trägt die Oszillationskomponente des Stimulationsstromfeldes zur Erzielung eines hohen Stimulationswirkungsgrades und Stimulationsvolumens bei, indem sie besonders in den Bereichen über den Elektroden wirkt, während die Rotationskomponente besonders im Bereich zwischen den Elektroden wirkt. Das Auftreten von lokal zu hohen Stromstärken, die zu Gewebeschädigungen führen könnten, wird vermieden.

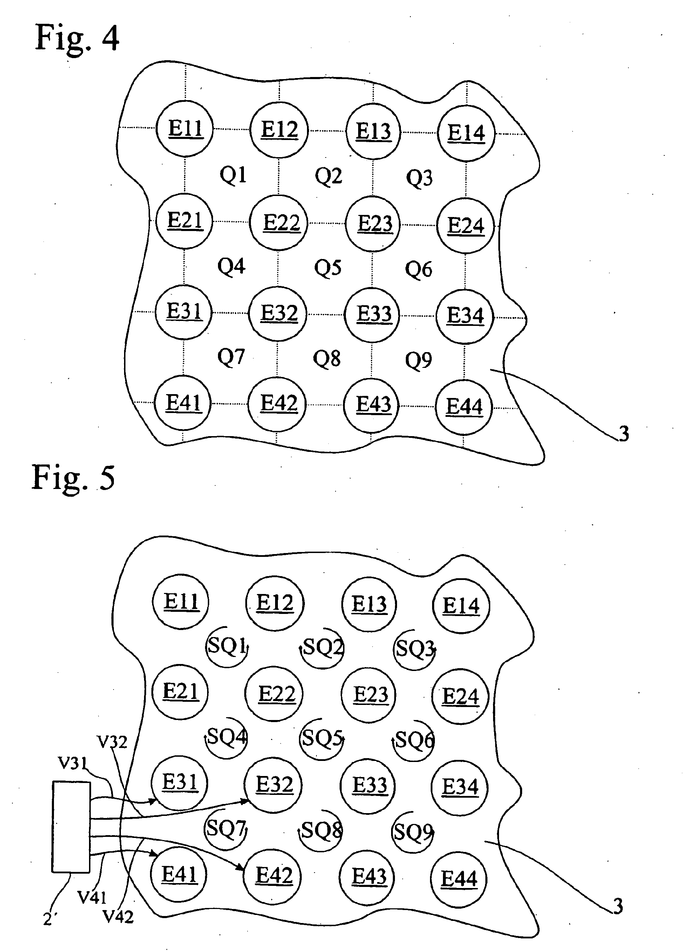

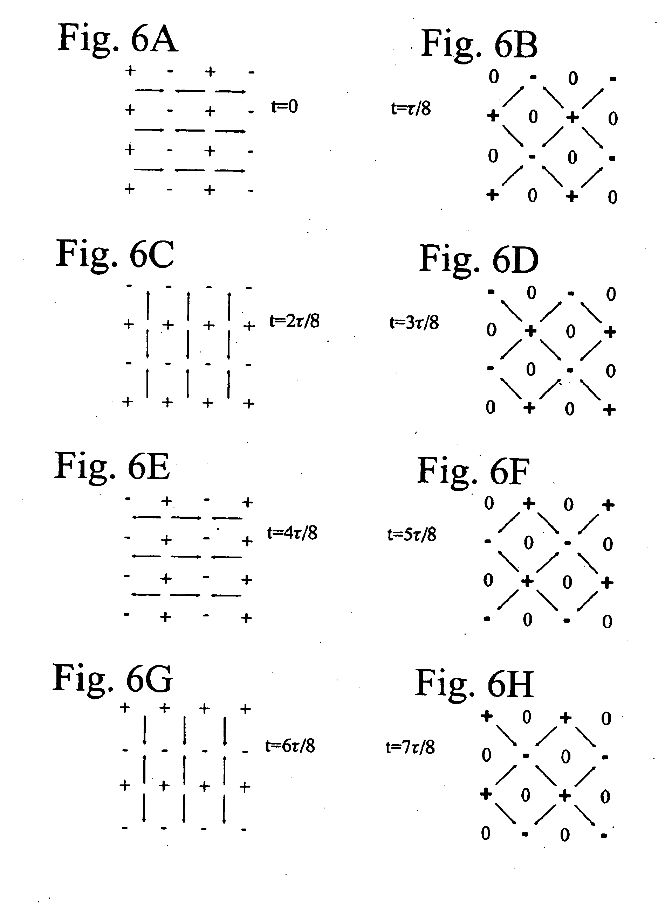

Die Fig. 4, 5 sowie 6A bis 6H veranschaulichen als weiteres Ausführungsbeispiel der Erfindung ein wiederum insbesondere als Retina-Implantat einsetzbares Mikro-/Multielektrodenarray 3 analog zum oben beschriebenen tripolaren Elektrodenarray, wobei hier jedoch die Einzelelektroden Eij in einem regelmäßigen Rechteckgitter angeordnet sind, bei dem es sich insbesondere um ein Quadratgitter handeln kann, mit einer Anzahl n von Zeilen und einer Anzahl m von Spalten, d.h. i=1, ... n und j = 1, ... m. Je vier benachbarte Elektroden bilden eine Quadrupol-Elementarzelle Q1, Q2, ..., indem sie entsprechend angesteuert werden. Da im Rechteckgitter jede innere Elektrode vier nächste Nachbarn hat, ist sie Teil von vier benachbarten Quadrupolen. Soweit nachstehend nichts anderes gesagt, entspricht dieses quadrupolare Multielektrodenarray in seiner Funktionsweise und seinen Eigenschaften und Vorteilen dem oben beschriebenen tripolaren Multielektrodenarray, worauf verwiesen werden kann. Dabei entsprechen die Fig. 4 der Fig. 1, die Fig. 5 der Fig. 2 und die Fig. 6A bis 6H den Fig. 3A bis 3F.

Speziell können im Stimulationsbetrieb die Einzelelektroden Eij des Rechteck-Elektrodengitters wiederum mit einer ansteuernden Wechselspannung Vij(t) der Form

$$Vij(t) = Iij(t) \cdot Bij(t) \cdot \sin(\omega t + Mij)$$

angesteuert werden, wobei die Bildmatrix Iij, die Signalformmatrix Bij und die Phasenmatrix Mij jeweils die gleiche Bedeutung haben wie oben zur tripolaren Anordnung erläutert. In Anpassung an das Rechteckgitter ist die Phasenmatrix Mij zwecks Quadrupolkopplung je vier benachbarter Einzelelektroden Eij verglichen mit dem obigen tripolaren Beispiel geeignet zu modifizieren. Eine mögliche Wahl für die Phasenmatrix Mij ist

$$Mij = (\pi/2) \cdot Sij + (3\pi/4),$$

wobei die Matrix Sij für den Quadrupolfall folgende Form hat:

$$Sij = \begin{pmatrix} 0 & 1 & 0 & 1 & 0 & 1 & . \\ -1 & 2 & -1 & 2 & -1 & 2 & . \\ 0 & 1 & 0 & 1 & 0 & 1 & . \\ -1 & 2 & -1 & 2 & -1 & 2 & . \\ 0 & 1 & 0 & 1 & 0 & 1 & . \\ -1 & 2 & -1 & 2 & -1 & 2 & . \\ . & . & . & . & . & . & . \end{pmatrix}$$

Wiederum kann jeder Quadrupol Q1, Q2, ... als ein Bildpunkt bzw. Pixel eines Retina-Implantats verstanden werden. Abgesehen von der oben zum tripolaren Beispiel erläuterten Modulation durch die zeitabhängige Bildmatrix Iij (t), welche die wahrzunehmende Bildinformation enthält, und durch die zeitabhängige Signalformmatrix Bij(t), welche vorzugsweise die Erzeugung von Kurzzeitpulsen bestimmt, ergibt sich aus den obigen Beziehungen die besagte Quadrupolansteuerung der Einzelelektroden Eij. Diese Ansteuerung kann wiederum von einer entsprechend ausgelegten Ansteuerspannungs-Bereitstellungseinheit 2' bewirkt werden, wie in Fig. 5 stellvertretend anhand der Ansteuerung der vier Elektroden E31, E32, E41 und E42 für den Quadrupol Q7 angedeutet. Wie in Fig. 5 schematisch dargestellt, wird im jeweiligen Quadrupolgebiet Q1, Q2, ... ein quadrupolares Stimulationsstromfeld SQ1, SQ2, ... mit Rotationskomponente erzeugt, d.h. die Stimulationsstromfeldlinien jedes Quadrupols Q1, Q2, ... rotieren im Quadrupolmittenbereich um die zur lokalen Normalenrichtung des Elektrodenarrays 3 parallele Quadrupolachse mit der Winkelgeschwindigkeit $\omega$ der Anregungspannung Vij. Dabei rotieren wiederum jeweils die Rotationskomponenten benachbarter Quadrupole gegensinnig. Es ergibt sich folglich auch für dieses Ausführungsbeispiel der gewünschte "Leuchtturmeffekt" von rotierenden Stimu-Iationsstromvektoren, hier für den Fall von lokalen Quadrupolfeldern.

Genauer ist dies wiederum aus den im Wesentlichen selbsterklärenden Momentandiagrammen der Fig. 6A bis 6H zu erkennen, in denen entsprechend zu den Fig. 3A bis 3F im obigen tripolaren Beispiel die Polarität und Stärke der momentanen Ansteuerspannung Vij am jeweiligen Gitterpunkt, d.h. der jeweiligen Einzelelektrode Eij, und die daraus resultierenden elektrischen Feldvektoren in der Mitte jedes Quadrupols in äquidistanten Zeitabständen einer Periode $\tau = 2\pi/\omega$ dargestellt sind, hier in Zeitabständen von $\Delta t = \tau/8$. Aus der Abfolge der schematischen Momentanaufnahmen ist wiederum ersichtlich, dass sich die zur Arrayebene parallele Komponente des angelegten elektrischen Feldes in der Mitte jedes Quadrupolgebietes Q1, Q2, ... mit der Winkelgeschwindigkeit $\omega$ der Ansteuerspannung Vij dreht und die Ansteuerspannung Vij für die verschiedenen Elektroden Eij so gewählt ist, dass das Ansteuerspannungssignal Vij für jede Elektrode Eij phasenrichtig zu jedem der vier Multipole beiträgt, dem jede innere Elektrode angehört. Dies resultiert in einem Stimulationsstrom-Gesamtfeld, zu dem alle Quadrupole Q1, Q2, ... konstruktiv in Form der einzelnen Quadrupol-Stromfelder SQ1, SQ2, ... mit Rotationskomponente beitragen, mit der erwähnten gegensinnigen Rotation jeweils benachbarter Quadrupol-Stromfelder SQ1, SQ2, ... Es sei der Vollständigkeit halber erwähnt, dass auch bei diesem Beispiel die Rotationskomponente in Quadrupolmitte am stärksten ausgeprägt ist und zu jeder der vier beteiligten Elektroden hin abnimmt und gleichzeitig der Rotationskomponente eine Oszillationskomponente überlagert ist, die umgekehrt über den Elektroden am stärksten ausgeprägt ist und zur Quadrupolmitte hin abnimmt.

Wenngleich sich die Darstellungen in den Fig. 6A bis 6H wiederum auf den vereinfachten Fall einer "weißen Wand" und Bij(t)=1 für alle i, j und t beziehen, wie oben zum tripolaren Fall erläutert, so bleibt dieses Resultat auch für den allgemeinen Fall qualitativ richtig. Mit anderen Worten, es ist auch in der Praxis möglich, mit der beschriebenen QuadrupolAnsteuerung der Elektroden Eij durch die entsprechenden Ansteuermittel 2 des Rechteck-Elektrodengitters Multipol-Stimulationsströme, hier speziell Quadrupol-Stimulationsströme, mit sich überlagernden Rotations- und Oszillationskomponenten im benachbarten biologischen Material zu erzeugen, wenn das Elektrodenarray 3 im Wirkbereich des biologischen Materials implantiert ist, z.B. als sub- oder epiretinales Sehhilfeimplantat. Es ergeben sich auch für die Quadrupolanordnung die oben zur Tripolanordnung beschriebenen Vorteile hinsichtlich Steigerung des Stimulationsvolumens und des Stimulationswirkungsgrades und Vermeidung von lokal zu hohen Strömen oder Spannungsabfällen. So ist es insbesondere auch bei der Quadrupolanordnung möglich, wie oben zur Tripolanordnung erläutert, die "Schussrichtung" der durch die Signalformmatrix Bij(t) festlegbaren Kurzzeitpulse entweder zu variieren oder festzuhalten und so den zusätzlichen Freiheitsgrad der Rotation zu einer günstigen Gestaltung des Stimulationssignals zu nutzen.

Wie die oben beschriebenen tripolaren und quadrupolaren Ausführungsbeispiele deutlich machen, stellt die Erfindung eine Vorrichtung zur elektrischen Stimulation von biologischem Material mit hohem Stimulationswirkungsgrad und großem Stimulationsvolumen zur Verfügung, wozu maßgeblich der Rotationseffekt der tripolaren oder höher multipolaren Stimulationsströme und auch deren Oszillationseffekt beiträgt. Zudem kann ein hohes Auflösungsvermögen erzielt werden, wenn eine Vielzahl kleinflächiger Einzelelektroden in einem Multielektrodenarray angeordnet werden. Durch die spezielle tripolare oder höher multipolare, kollektiv zusammenwirkende Ansteuerung der Einzelelektroden werden Probleme herkömmlicher monopolarer und bipolarer Anordnungen weitestgehend vermieden. Insbesondere treten auch bei relativ großer Bildhelligkeit bis hin zum Fall einer "weißen Wand" und hoher Elektrodenintegrationsdichte keine störenden kollektiven Stimulationsstromeffekte, wie übermäßig hohe Spannungsabfälle etc. , auf.

Wie die oben erläuterten Ausführungsbeispiele deutlich machen, ermöglicht die Erfindung insbesondere die Erzeugung von Stimulationsfeldern als Multipolarray, bei dem die Stimulationsfeldbeiträge von Nächste-Nachbarn-Multipolen gleichzeitig, aber mit entgegengesetztem Drehsinn rotieren können. Es versteht sich, dass in alternativen Ausführungsformen durch modifizierte Ansteuerung der Einzelelektroden auch Stimulationsfelder mit anderen Mustern rotierender Multipolfelder erzeugt werden können. Insbesondere umfasst die Erfindung auch Ausführungsformen mit zeitlich alternierender bzw. gepulster Ansteuerung der aus jeweils drei oder mehr benachbarten Einzelelektroden gebildeten Multipol-Elementarzellen, so dass bei Bedarf z.B. auch ein Stimulationsfeld erzeugt werden kann, bei dem die Stimulationsfelder benachbarter Multipol-Elementarzellen gleichsinnig, aber zeitlich versetzt rotieren.

**Patentansprüche**

1. Vorrichtung zur elektrischen Stimulation von elektrisch stimulierbarem biologischem Material, insbesondere von Netzhautmaterial, mit

   - einem in Wirkkontakt zu dem biologischen Material implantierbaren Elektrodenarray (1) mit einer zweidimensionalen Anordnung von Einzelelektroden (Eij), die bei Beaufschlagung mit einem Stimulationssignal (Vij) ein Stimulationsfeld (S1, S2, ...) für das biologische Material erzeugen, und
   - Mitteln (2) zur Beaufschlagung der Einzelelektroden mit den Stimulationssignalen, wobei die Elektrodenbeaufschlagungsmittel (2) zur Beaufschlagung der Einzelelektroden (Eij) mit Wechselfeld-Stimulationssignalen (Vij) derart eingerichtet sind, dass das Elektrodenarray (1) wenigstens zwei tripolare oder höher multipolare Multipol-Elementarzellen (T1, T2, ...; Q$_1$, Q$_2$, ...) aus jeweils drei oder mehr benachbarten Einzelelektroden bildet,
   **dadurch gekennzeichnet, dass**
   - das von jeder Multipol-Elementarzelle für das biologische Material erzeugte Stimulationsfeld (S1, S2, ...) eine Rotationskomponente aufweist und wenigstens eine der Einzelelektroden zu wenigstens zwei Multipol-Elementarzellen gehört.

2. Vorrichtung nach Anspruch 1, weiter **dadurch gekennzeichnet, dass** das von der jeweiligen Multipol-Elementarzelle für das biologische Material erzeugte Stimulationsfeld eine um eine Multipolachse rotierende Feldkomponente aufweist.

3. Vorrichtung nach Anspruch 1 oder 2, weiter **dadurch gekennzeichnet, dass** die Wechselfeld-Stimulationssignale für die Einzelelektroden der Multipol-Elementarzelle phasenverschobene Anteile eines Wechselspannungssignals beinhalten.

4. Vorrichtung nach einem der Ansprüche 1 bis 3, weiter **dadurch gekennzeichnet, dass** die Einzelelektroden in einem regelmäßigen Gitter angeordnet sind, das mehrere nebeneinanderliegende Multipol-Elementarzellen bildet, wobei jede innere Einzelelektrode der Gitteranordnung zu mehreren Multipol-Elementarzellen gehört.

5. Vorrichtung nach Anspruch 4, weiter **dadurch gekennzeichnet, dass** die Einzelelektroden in einem regelmäßigen Dreieckgitter oder einem regelmäßigen Viereckgitter angeordnet sind.

6. Vorrichtung nach einem der Ansprüche 3 bis 5, weiter **dadurch gekennzeichnet, dass** die Wechselfeld-Stimulationssignale einen zusätzlichen multiplikativen Bildinformationsanteil und einen zusätzlichen multiplikativen Signalformanteil beinhalten.

7. Vorrichtung nach einem der Ansprüche 1 bis 6, weiter **dadurch gekennzeichnet, dass** sie als Retina-Implantat ausgelegt ist und das Elektrodenarray ein retina-implantierbares Mikroelektrodenarray ist, bei dem die Einzelelektroden sämtlich auf einer Seite eines Trägerchips angeordnet sind.

8. Vorrichtung nach einem der Ansprüche 4 bis 7, weiter **dadurch gekennzeichnet, dass** die Multipol-Elementarzellen von den Elektrodenbeaufschlagungsmitteln derart mit den Wechselfeld-Stimulationssignalen beaufschlagbar sind, dass die Stimulationsfelder der verschiedenen Multipol-Elementarzellen gleichzeitig rotierende Feldkomponenten mit gegensinnigem Drehsinn benachbarter Multipol-Elementarzellen aufweisen.

## Claims

1. Device for electrically stimulating biological material that can be electrically stimulated, in particular retinal material, comprising

   - an electrode array (1) which can be implanted in active contact with the biological material and includes a two-dimensional arrangement of individual electrodes (Eij), which upon actuation with a stimulation signal (Vij) produce a stimulation field (S1, S2,...) for the biological material, and
   - means (2) for actuating the individual electrodes with the stimulation signals, wherein the electrode actuating means (2) for actuating the individual electrodes (Eij) with alternating field stimulation signals (Vij) are configured such that the electrode array (1) forms at least two tripolar or higher multipolar multipole elementary cells (T1, T2 ...; Q1, Q2,...) from three or more adjacent individual electrodes, respectively,
   **characterized in that**
   - the stimulation field (S1, S2, ...) produced by every multipole elementary cell for the biological material exhibits a rotation component, and at least one of the individual electrodes belongs to at least two multipole elementary cells.

2. Device according to claim 1, further **characterized in that** the stimulation field, produced by the respective multipole elementary cells for the biological material, exhibits a field component, which rotates about a multipole axis.

3. Device according to claim 1 or 2, further **characterized in that** the alternating field stimulation signals for the individual electrodes of the multipole elementary cell include phase-shifted components of an alternating voltage signal.

4. Device according to any of claims 1 to 3, further **characterized in that** the individual electrodes are arranged in a regular lattice, which forms a plurality of multipole elementary cells, which lie side by side, each internal individual electrode of the lattice structure belonging to a plurality of multipole elementary cells.

5. Device according to claim 4, further **characterized in that** the individual electrodes are arranged in a regular triangular lattice or a regular rectangular lattice.

6. Device according to any of claims 3 to 5, further **characterized in that** the alternating field stimulation signals include an additional multiplicative image information component and an additional multiplicative signal shape component.

7. Device according to any of claims 1 to 6, further **characterized in that** it is designed as a retina implant and that the electrode array is a microelectrode array which can be implanted in a the retina and in which the individual electrodes are all arranged on one side of a carrier chip.

8. Device according to any of claims 4 to 7, further **characterized in that** the multipole elementary cells are actuated by the electrode actuating means with the alternating field stimulation signals such that the stimulation fields of the various multipole elementary cells exhibit simultaneously rotating field components having opposite rotation directions of adjacent multipole elementary cells.

**Revendications**

1. Dispositif pour la stimulation électrique de matériel biologique stimulable électriquement, en particulier de matériel rétinien, avec

   - une batterie d'électrodes (1) implantable en contact actif avec le matériel biologique, avec un agencement en deux dimensions d'électrodes individuelles (Eij), qui génèrent un champ de stimulation (S 1, S2, ...) pour le matériel biologique en cas d'alimentation avec un signal de stimulation (Vij,) et
   - des moyens (2) pour l'alimentation des électrodes individuelles avec les signaux de stimulation, les moyens d'alimentation des électrodes (2) pour l'alimentation des électrodes individuelles (Eij) avec des signaux de stimulation à champ alternatif (Vij) étant aménagés de manière à ce que la batterie d'électrodes (1) forme au moins deux cellules élémentaires multipôles tripolaires ou comprenant un nombre de pôles plus élevé (T1, T2, ... $Q_1$' $Q_2$, ...) à partir de trois ou plus de trois électrodes individuelles voisines,
   **caractérisé en ce que**
   - le champ de stimulation (S1, S2, ...) généré par chaque cellule élémentaire multipôle pour le matériel biologique présente une composante de rotation et qu'au moins l'une des électrodes individuelles fait partie d'au moins deux cellules élémentaires multipôles.

2. Dispositif suivant la revendication 1, **caractérisé en outre en ce que** le champ de stimulation généré par la cellule élémentaire multipôle concernée pour le matériel biologique présente une composante de champ tournant autour d'un axe multipôle.

3. Dispositif suivant la revendication 1 ou 2, **caractérisé en outre en ce que** les signaux de stimulation à champ alternatif comprennent pour les électrodes individuelles de la cellule élémentaire multipôle des parties décalées en phase d'un signal à tension alternative.

4. Dispositif suivant l'une des revendications 1 à 3, **caractérisé en outre en ce que** les électrodes individuelles sont disposées dans une grille régulière, qui forme plusieurs cellules élémentaires multipôles adjacentes, chaque électrode individuelle interne de l'agencement de grille faisant partie de plusieurs cellules élémentaires multipôles.

5. Dispositif suivant la revendication 4, **caractérisé en outre en ce que** les électrodes simples sont disposées dans une grille triangulaire régulière ou dans une grille rectangulaire régulière.

6. Dispositif suivant l'une des revendications 3 à 5, **caractérisé en outre en ce que** les signaux de stimulation à champ alternatif contiennent une part d'information d'image multiplicative supplémentaire et une part de formation de signal multiplicative supplémentaire.

7. Dispositif suivant l'une des revendications 1 à 6, **caractérisé en outre en ce qu'**il est agencé comme un implant de rétine et que la batterie d'électrodes est une batterie de microélectrodes implantables dans la rétine pour laquelle les électrodes individuelles sont disposés globalement sur une face de la puce porteuse.

8. Dispositif suivant l'une des revendications 4 à 7, **caractérisé en outre en ce que** les cellules élémentaires multipôles peuvent être alimentées par des moyens d'alimentation d'électrodes de façon telle avec les signaux de stimulation à champ alternatif que les champs de stimulation des différentes cellules élémentaires multipôles présentent des composantes de champ de rotation simultanément avec des cellules élémentaires multipôles voisines à sens de rotation opposé.

EP 1 871 466 B1

## Fig. 1

## Fig. 2

## Fig. 3A

$t=0$

## Fig. 3B

$t=\tau/6$

## Fig. 3C

$t=2\tau/6$

## Fig. 3D

$t=3\tau/6$

## Fig. 3E

$t=4\tau/6$

## Fig. 3F

$t=5\tau/6$

## Fig. 4

## Fig. 5

# Fig. 6A

$t=0$

# Fig. 6B

$t=\tau/8$

# Fig. 6C

$t=2\tau/8$

# Fig. 6D

$t=3\tau/8$

# Fig. 6E

$t=4\tau/8$

# Fig. 6F

$t=5\tau/8$

# Fig. 6G

$t=6\tau/8$

# Fig. 6H

$t=7\tau/8$

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10151650 A1
- DE 10329615 A1
- DE 19705987 C2
- US 20020091422 A

**In der Beschreibung aufgeführte Nicht-Patentliteratur**

- **J.F. Rizzo III et al.** Methods and perceptual thresholds for short-term electrical stimulation of human retina with microelectrode arrays. *Investigative Ophthalmology & Visual Science,* Dezember 2003, vol. 44, 5355